# EUROPEAN PATENT APPLICATION

(11) **EP 4 032 534 A1**
(43) Date of publication of application: **27.07.2022**
(21) Application number: 21152993.8
(22) Date of filing: 22.01.2021
(51) Int. Cl.: A61K 31/4545, A61K 45/06, A61K 39/00, A61P 31/14

(54) **LONAFARNIB FOR USE IN THE TREATMENT OF VIRAL INFECTIONS**

(71) Applicant: Twincore Zentrum für Experimentelle und Klinische Infektionsforschung GmbH, 30625 Hannover (DE); Medizinische Hochschule Hannover, 30625 Hannover (DE)
(72) Inventor: SAKE, Svenja, 30655 Hannover (DE); HAID, Sibylle, 30169 Hannover (DE); RÜCKERT, Jessica, 30625 Hannover (DE); SCHULZ, Thomas, 30625 Hannover (DE); PIETSCHMANN, Thomas, 30625 Hannover (DE)
(74) Representative: Kuttenkeuler, David

(57) **Abstract**

The invention pertains to the use of lonafarnib or any analog or derivative thereof for the treatment of a viral disease characterized by a viral entry into a host cell via a viral fusion glycoprotein (F-protein). In particular, the invention provides treatments of human respiratory syncytial virus (HRSV) caused diseases comprising the administration (use) of lonafarnib or its analogs or derivatives, as well as pharmaceutical compositions comprising lonafarnib or its analogs and derivates.

## Description

### FIELD OF THE INVENTION

The invention pertains to the use of lonafarnib or any analog or derivative thereof for the treatment of a viral disease characterized by a viral entry into a host cell via a viral fusion glycoprotein (F-protein). In particular, the invention provides treatments of human respiratory syncytial virus (HRSV) caused diseases comprising the administration (use) of lonafarnib or its analogs or derivatives, as well as pharmaceutical compositions comprising lonafarnib or its analogs and derivates.

### DESCRIPTION

The human respiratory syncytial virus (HRSV) is a human pathogenic RNA virus that can cause infections of the upper and lower respiratory tract. The virus is spread worldwide and causes recurring epidemics every year. The course of infection is predominantly mild. However, there are also severe infections involving the lower respiratory tract that can be fatal. Risk groups for severe courses of infection are infants (especially preterm babies), the elderly and people with weakened immune systems. In the group of infants under 5 years of age alone, there are about 3.4 million severe HRSV infections annually, causing up to 199,000 deaths (1).

The HRSV infection is currently being treated symptomatically. Direct antiviral therapy is not approved. Only ribavirin, a broad antiviral nucleoside, is approved for use. However, due to considerable side effects and lack of effectiveness, it is hardly ever used clinically for the treatment of HRSV. In addition, a monoclonal antibody (palivizumab) against the HRSV fusion protein F is available. This antibody is mainly used for the prophylaxis of a severe HRSV infection in the first year of life of children with risk factors for severe courses. The virus can develop resistance to palivizumab. Other HRSV inhibitors, including various cell entry inhibitors, are in pre-clinical and clinical development (2).

Thus, it is an object of the invention to provide further therapeutics for the treatment of diseases associated with an infection of HRSV.

### BRIEF DESCRIPTION OF THE INVENTION

The object of the invention is solved by the use of lonafarnib for the treatment of viral diseases such as diseases caused by HRSV. The inventors were able to show the antiviral effect of lonafarnib against HRSV. In addition, the present invention discloses mechanism of action of lonafarnib against HRSV. Further, the inventors were able to show that lonafarnib has a direct antiviral effect on HRSV by disrupting the function of the HRSV F protein.

These new findings position lonafarnib for use according to the present invention as a direct antiviral molecule for the treatment of HRSV infections in humans.

Generally, and by way of brief description, the main aspects of the present invention can be described as follows:

In **a first aspect,** the invention pertains to a compound for use in the treatment of a viral disease in a subject, wherein the viral disease is caused by a virus comprising a membrane fusion glycoprotein (F-protein), wherein the compound is lonafarnib or a derivative thereof, or solvates, salts, stereoisomers, complexes, polymorphs, crystalline forms, racemic mixtures, diastereomers, enantiomers, tautomers, isotopically labelled forms, prodrugs, and combinations thereof.

In **a second aspect,** the invention pertains to a pharmaceutical composition for use in the treatment of a viral disease in a subject, wherein the viral disease is caused by a virus comprising a membrane fusion glycoprotein (F-protein), wherein the pharmaceutical composition comprises lonafarnib or a derivative thereof, or solvates, salts, stereoisomers, complexes, polymorphs, crystalline forms, racemic mixtures, diastereomers, enantiomers, tautomers, isotopically labelled forms, prodrugs, and combinations thereof, and a pharmaceutically acceptable carrier and/or excipient.

In **a third aspect,** the invention pertains to a method for inhibiting entry of a virus into a host cell, comprising contacting the virus with lonafarnib or a derivative thereof.

### DETAILED DESCRIPTION OF THE INVENTION

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a compound for use in the treatment of a viral disease in a subject, wherein the viral disease is caused by a virus comprising a membrane fusion glycoprotein (F-protein), wherein the compound is lonafarnib or a derivative thereof, or solvates, salts, stereoisomers, complexes, polymorphs, crystalline forms, racemic mixtures, diastereomers, enantiomers, tautomers, isotopically labelled forms, prodrugs, and combinations thereof.

In another alternative aspect of the invention a method for treating a viral disease in a subject is provided, comprising administering lonafarnib a derivative thereof, or solvates, salts, stereoisomers, complexes, polymorphs, crystalline forms, racemic mixtures, diastereomers, enantiomers, tautomers, isotopically labelled forms, prodrugs, and combinations thereof to the subject. Alternatively, a use of lonafarniba derivative thereof, or solvates, salts, stereoisomers, complexes, polymorphs, crystalline forms, racemic mixtures, diastereomers, enantiomers, tautomers, isotopically labelled forms, prodrugs, and combinations thereof in the manufacture of a medicament for the treatment of a viral disease is disclosed.

The term "lonafarnib,"' also known under the trade name Sarasar^{®} (Schering), refers to a farnesyltransferase (FTase) inhibitor, 4(2[4-[(11R)-3,10-dibromo-8-chloro-6,11-dihydro-5Hbenzo[5,6]-cyclohepta[1,2b]pyridin-nyl]-piperidino]-2-oxoethyl]-1-piperidinecarboxamide), which has the structure (I) shown below:

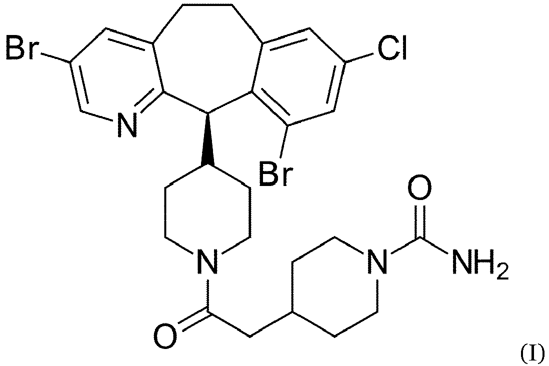

Lonafarnib is a solid with a melting point of approximately 200° C. and is non-hygroscopic. Its molecular weight is 638.7. In the solid state, the compound is thermally stable. In solution, it is stable at neutral pH but will hydrolyze in acidic or basic conditions. It is a poorly water soluble tricyclic compound, which when formulated in crystalline forms results in low and variable bioavailability in animals. In pharmaceutical dosage forms, lonafarnib may be administered in the form of its pharmaceutically acceptable salts, or pharmaceutically acceptable solvates of lonafarnib and its salts, or may be used alone or in appropriate association, as well as in combination, with other pharmaceutically active compounds. Specific dosage forms of lonafarnib are described herein below in the section relating to pharmaceutical compositions.

The term "F protein" or "fusion protein" or "fusion glycoprotein" refers to a polypeptide or protein of an enveloped virus, which during the process of reproduction of the virus mediates fusion of the viral membrane with respective host membranes to deliver their genome into the host cell. The attachment to the host cell receptors as well as fusion with the target membrane is mediated by F proteins. Preferably, the F protein of the invention is a protein having all or part of an amino acid sequence of an HRSV Fusion protein polypeptide. An F-protein, or a homolog or paralog thereof, is preferably a protein comprising an amino acid sequence that is at least 50%, 60%, 70%, 80%, 85%, 90%, 95%, 96%, 97%, 98%, 99% or 100% identical to the sequence shown in any one of SEQ ID NO: 1 to 3 which depict fusion glycoprotein Fo, F1 and F2 of Human respiratory syncytial virus A (strain A2) (see also UniProtKB - P03420). Alternative is an F-protein of HRSV-B. Such Fo protein is an inactive precursor protein that is cleaved at two sites by a furin-like protease to give rise to the mature F1 (SEQ ID NO: 2) and F2 (SEQ ID NO: 3) fusion glycoproteins.

In preferred embodiments the invention provides a treatment which involves inhibition of an F-protein mediated entry of the virus into a host cell. Without being bound to theory, surprisingly the compounds of the invention have antiviral activity by inhibiting the fusion process of enveloped viruses such as HRSV to their host cells.

Preferably a treatment in accordance with the invention comprises the administration of a therapeutically effective amount of the compound to a subject in need of the treatment, preferably wherein the subject is suspected to suffer from the viral disease. The term "therapeutically effective amount" as used herein refers to that amount of an embodiment of the agent (which may be referred to as a compound, an inhibitory agent, and/or a drug, preferably of lonafarnib) being administered that will treat to some extent a disease, disorder, or condition, e.g., relieve one or more of the symptoms of the disease, i.e., infection, being treated, and/or that amount that will prevent, to some extent, one or more of the symptoms of the disease, i.e., infection, that the subject being treated has or is at risk of developing.

The terms "treatment", "treating", and "treat" are defined as acting upon a disease, disorder, or condition with an agent to reduce or ameliorate the pharmacologic and/or physiologic effects of the disease, disorder, or condition and/or its symptoms. "Treatment," as used herein, covers any treatment of a disease in a human subject, and includes: (a) reducing the risk of occurrence of the disease in a subject determined to be predisposed to the disease but not yet diagnosed as infected with the disease, (b) impeding the development of the disease, and/or (c) relieving the disease, i.e., causing regression of the disease and/or relieving one or more disease symptoms. "Treatment" is also meant to encompass delivery of an inhibiting agent to provide a pharmacologic effect, even in the absence of a disease or condition. For example, "treatment" encompasses delivery of a disease or pathogen inhibiting agent that provides for enhanced or desirable effects in the subject (e.g., reduction of pathogen viral load, reduction of disease symptoms, etc.).

The virus in accordance with the present invention is preferably an enveloped virus. Such viruses can be selected from the group of orthopneumoviruses, and preferably is a human respiratory syncytial virus (HRSV). Such HRSV strains according to the invention may be HRSV-A or HRSV-B.

A "disease" in context of the present invention means any disease caused, directly or indirectly, by an infection with a virus, such as HRSV, as well as diseases or conditions which predispose a patient to infection by a virus, such as HRSV. Examples of diseases falling into the former category include pneumonia and bronchiolitis. Diseases and conditions in the latter category (i.e., those which place the patient at risk of severe viral infection, in particular HRSV infection) include cystic fibrosis, congenital heart disease, cancer, age related immunosuppression, transplant recipients and, generally, any condition that causes a state of immunosuppression or decreased function of the immune system such as postoperative organ transplantation regimens or premature birth.

In context of the invention the viral disease is preferably an HRSV infection of the lower or upper respiratory tract of the subject.

Furthermore, it is preferred that the subject to be treated in accordance with the invention belongs to one or more risk group indicated for a fatal disease course selected from
- Children between o and 10 years of age;
- Elderly of 50 years of age or older;
- Immunosuppressed patients (see above).

Treatments in accordance with the herein disclosed invention in addition may comprise the administration of one or more additional therapeutic effective for treating the viral disease, such compound being selected from antiviral compounds and compounds ameliorating one or more symptoms of the viral disease. Preferred examples of the antiviral compound are selected from ribavirin, Palivizumab, MK-1654, MEDI8897, JNJ-53718678, Ziresovir, RV521, Lumicitabine and EDP-938. A preferred additional therapeutic is Palivizumab.

In this context, the subject to be treated is preferably suffering from an infection with a virus which is a HRSV with a resistance to treatment with Palivizumab. Farnesyl transferase in context of the invention is an enzyme that adds a 15-carbon isoprenoid called a farnesyl group to proteins bearing a four-amino acid sequence at the carboxyl terminus of a protein.

Surprisingly it was found in context of the invention that the therapeutic action of lonafarnib in the treatment of HRSV infections, and similar viral infections, is not dependent on the known function of lonafarnib as inhibitor of farnesyl transferases. Hence, preferably the treatment does not involve inhibition of farnesyl transferases, and/or is independent of farnesyl transferase activity. Hence, the viral disease of the invention is preferably one that is not treatable by an inhibition of a farnesyltransferase, preferably the viral disease is not hepatitis, and most preferably not hepatitis D.

A treatment of the invention may comprise the administration of the compound at a daily dose of 75 - 150 mg per day, for example 75 mg per day, 100 mg per day, or 150 mg per day.

A treatment may be a preventive treatment of a subject suspected to get into contact and be infected with the virus.

In **a second aspect,** the invention pertains to a pharmaceutical composition for use in the treatment of a viral disease in a subject, wherein the viral disease is caused by a virus comprising a membrane fusion glycoprotein (F-protein), wherein the pharmaceutical composition comprises lonafarnib or a derivative thereof, or solvates, salts, stereoisomers, complexes, polymorphs, crystalline forms, racemic mixtures, diastereomers, enantiomers, tautomers, isotopically labelled forms, prodrugs, and combinations thereof, and a pharmaceutically acceptable carrier and/or excipient.

The term "pharmaceutical composition" is meant to encompass a composition suitable for administration to a subject. In general a "pharmaceutical composition" is sterile, and preferably free of contaminants that are capable of eliciting an undesirable response within the subject (e.g., the compound(s) in the pharmaceutical composition is pharmaceutical grade). Pharmaceutical compositions can be designed for administration to subjects or patients in need thereof via a number of different routes of administration including oral, intravenous, buccal, rectal, parenteral, intraperitoneal, intradermal, intratracheal, intramuscular, subcutaneous, inhalational and the like. Preferably, the compound is administered orally, intravenously or via a respiratory route, for example by oral or nasal inhalation.

The terms "pharmaceutically acceptable excipient," "pharmaceutically acceptable diluent," "pharmaceutically acceptable carrier," or "pharmaceutically acceptable adjuvant" means an excipient, diluent, carrier, and/or adjuvant that are useful in preparing a pharmaceutical composition that are generally safe, non-toxic and neither biologically nor otherwise undesirable, and include an excipient, diluent, carrier, and adjuvant that are acceptable for veterinary use and/or human pharmaceutical use. "A pharmaceutically acceptable excipient, diluent, carrier and/or adjuvant" as used in the specification and claims includes one and more such excipients, diluents, carriers, and adjuvants. A wide variety of pharmaceutically acceptable excipients, such as vehicles, adjuvants, carriers or diluents, and auxiliary substances, such as pH adjusting and buffering agents, tonicity adjusting agents, stabilizers, wetting agents and the like, are known in the art. Pharmaceutically acceptable excipients have been amply described in a variety of publications, including, for example, A. Gennaro (2000) "Remington: The Science and Practice of Pharmacy," 20th edition, Lippincott, Williams, & Wilkins; Pharmaceutical Dosage Forms and Drug Delivery Systems (1999) H.C. Ansel et al., eds., 7th ed., Lippincott, Williams, & Wilkins; and Handbook of Pharmaceutical Excipients (2000) A.H. Kibbe et al., eds., 3rd ed. Amer. Pharmaceutical Assoc. For oral preparations, lonafarnib can be used alone or in pharmaceutical formulations of the invention comprising, or consisting essentially of, or consisting of lonafarnib in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The term "pharmaceutically acceptable salt" refers to those salts that retain the biological effectiveness and optionally other properties of the free bases and that are obtained by reaction with inorganic or organic acids such as hydrochloric acid, hydrobromic acid, sulfuric acid, nitric acid, phosphoric acid, methanesulfonic acid, ethanesulfonic acid, p- toluenesulfonic acid, salicylic acid, malic acid, maleic acid, succinic acid, tartaric acid, citric acid, and the like. In the event that embodiments of the disclosed agents form salts, these salts are within the scope of the present disclosure. Reference to an agent of any of the formulas herein is understood to include reference to salts thereof, unless otherwise indicated.

The term "unit dosage form," as used herein, refers to physically discrete units suitable as unitary dosages for human subjects, each unit containing a predetermined quantity of a compound (e.g., an anti-viral compound, as described herein) or compounds, calculated in an amount sufficient to produce the desired treatment effect in association with a pharmaceutically acceptable diluent, carrier or vehicle.

The term "oral dosage form," as used herein, refers to a dosage form that is orally administered such as tablets, capsules, gel caps, syrups, elixirs, and suspensions. "Solid oral dosage forms" include tablets, capsules, caplets, and the like.

The term "oral unit dosage form," as used herein, refers to a unit dosage form that is that orally administered. The term "respiratory route" as used herein refers to both nasal and pulmonary respiratory routes, and includes for example dosage forms for inhalation such as sprays and the like.

All deuterated analogs (a compound is a deuterated analog of another compound, the "parent compound", if it differs from the parent compound by only replacement of one or more hydrogen atoms with one or more deuterium atoms) of any active pharmaceutical ingredient described herein, including without limitation, lonafarnib, or additional compounds, are, for purposes of the present invention, encompassed by reference to the parent compound.

All stereoisomers of any agent described herein, including without limitation, lonafarnib, ritonavir, cobicistat, and any other active pharmaceutical agent described herein, such as those that may exist due to asymmetric carbons on the various substituents, including enantiomeric forms (which may exist even in the absence of asymmetric carbons) and diastereomeric forms, are contemplated within the scope of this disclosure. Individual stereoisomers of the compounds of the disclosure may, for example, be substantially free of other isomers, or may be admixed, for example, as racemates or with all other, or other selected, stereoisomers. The stereogenic centers of the compounds of the present disclosure can have the S or R configuration as defined by the lUPAC 1974 Recommendations.

In some embodiments, the unit dosage form or pharmaceutical composition comprises 25, 50, 75, or 100 mg lonafarnib or a pharmaceutically acceptable salt thereof. In some embodiments, the unit dosage form or pharmaceutical composition comprises 25 mg lonafarnib or a pharmaceutically acceptable salt thereof. In some embodiments, the unit dosage form or pharmaceutical composition comprises 50 mg lonafarnib or a pharmaceutically acceptable salt thereof. In some embodiments, the unit dosage form or pharmaceutical composition comprises 75 mg lonafarnib or a pharmaceutically acceptable salt thereof. In some embodiments, the unit dosage form or pharmaceutical composition comprises 100 mg lonafarnib or a pharmaceutically acceptable salt thereof.

In **a third aspect,** the invention pertains to a method for inhibiting entry of a virus into a host cell, comprising contacting the virus with lonafarnib or a derivative thereof.

Such method may be an in-vivo or ex-vivo (in-vitro) method.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

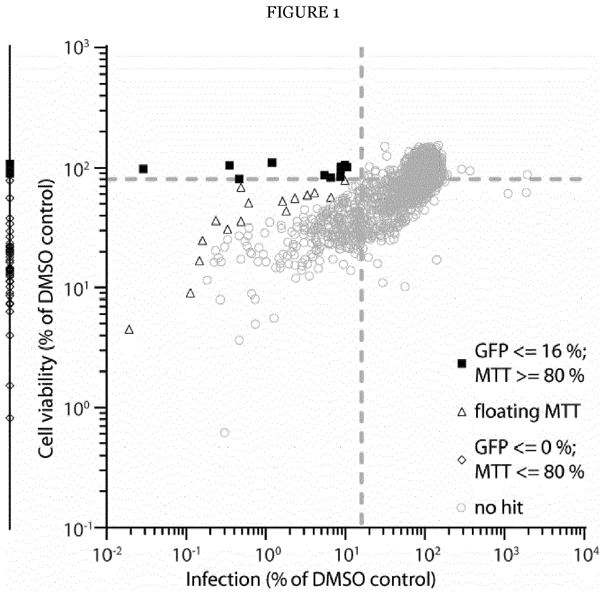

**Figure 1**: shows the primary screening of the ReFRAME library with an HRSV A GFP reporter virus and hit selection. The dotted lines indicate the thresholds of the inclusion criteria (infection signal [GFP] < 16% and cell viability [MTT] > 80%). Additional hits were also included in the MTT assay due to a floating MTT threshold. Each point represents one compound. The symbol shape indicates whether a compound was defined and followed up as a "hit" (black square (hit according to classical inclusion criteria); white triangle with black frame (hit after floating inclusion threshold), white diamond with black frame (selected for follow up due to GFP signal similar to uninfected wells; cell viability is depicted on line next to the graph)white circle with grey frame (no hit). Mean values of a double determination are shown.

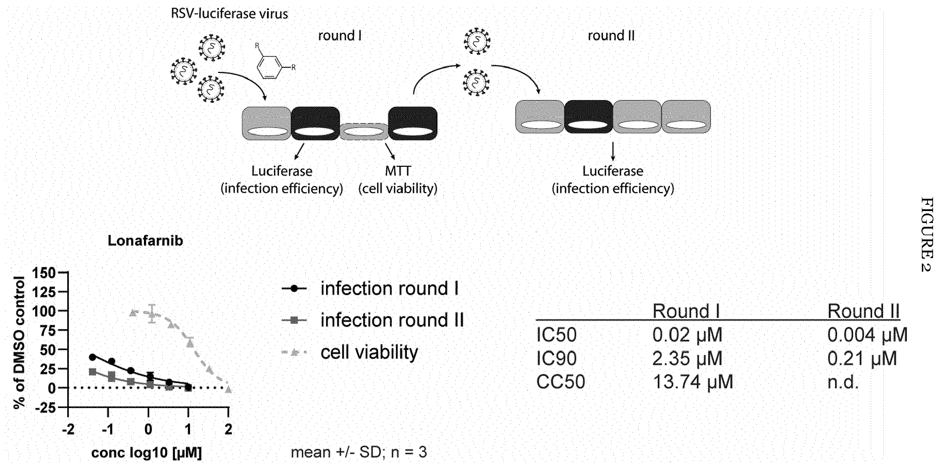

**Figure 2**: shows that the farnesyltransferase inhibitor lonafarnib is an effective inhibitor of HRSV infection in cell culture. The schematic drawing shows the implementation of the infection experiment with the HRSV luciferase reporter virus. The dose-response relationship of lonafarnib to the HRSV reporter virus is shown on the right. Below are the IC50 and IC90 values of the first round of infection and the second round of infection, respectively, and the 50% cytotoxic concentration of lonafarnib (CC50).

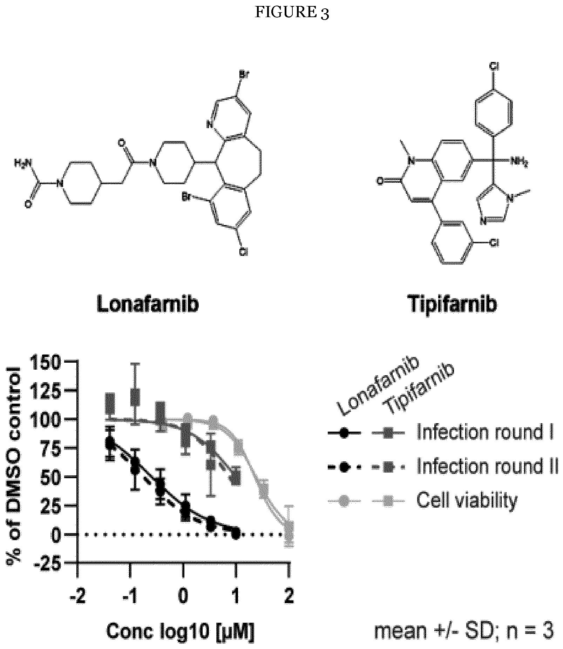

**Figure 3**: shows a comparative analysis of the antiviral effect of two chemically different farnesyltransferase inhibitors (lonafarnib and tipifarnib) against HRSV. The chemical structure of the farneslytransferase inhibitors lonafarnib and tipifarnib is shown above. The dose-response relationship of these agents against the HRSV luciferase reporter virus is shown below.

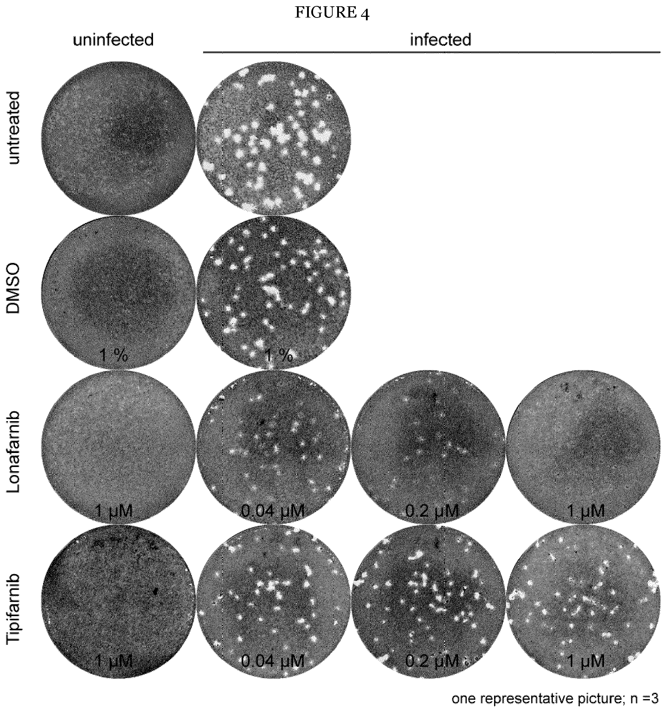

**Figure 4****:** shows that lonafarnib but not tipifarnib reduces plaque formation of a clinical HHRSV ON1 isolate. The respective concentrations of lonafarnib, tipifarnib and DMSO (control; final concentration 1%) administered to the cells are indicated on the respective well.

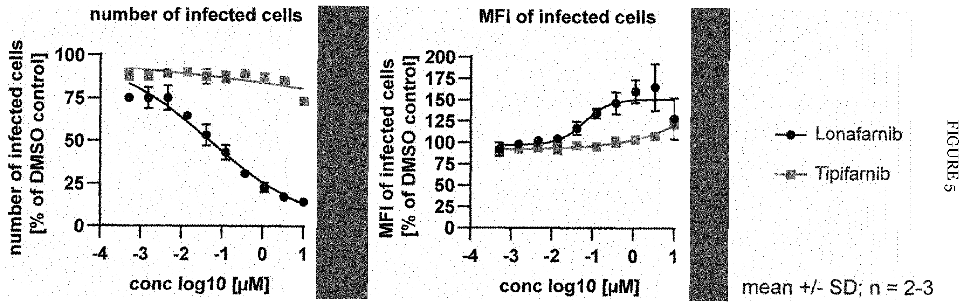

**Figure 5****:** shows that lonafarnib reduces number but not viral load of cells infected with clinical HHRSV ON1 isolate dose dependently. Both graphs show data from analysing the viral infection efficiency by intracellular flow cytometry staining against a virus own protein. Left graph: Shows dose-dependent reduction of the number of infected cells with increasing administration of lonafarnib. Right graph: Shows no reduction of virus proteins in infected cells. MFI: mean fluorescence intensity

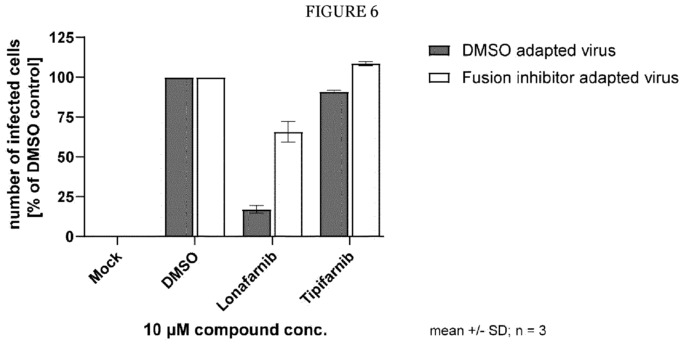

**Figure 6****:** shows that lonafarnib has reduced activity against a population of HRSV viruses that have previously developed resistance to an HRSV fusion inhibitor by way of mutations within the F protein. The graph shows the analysis of the effectiveness of HRSV infection (corresponding to the number of infected cells) determined by intracellular flow cytometry staining. The most significant reduction of infected cells is shown in the DMSO adapted virus population by administration of lonafarnib. Dark grey: DMSO adapted virus; light grey: fusion inhibitor adapted virus.

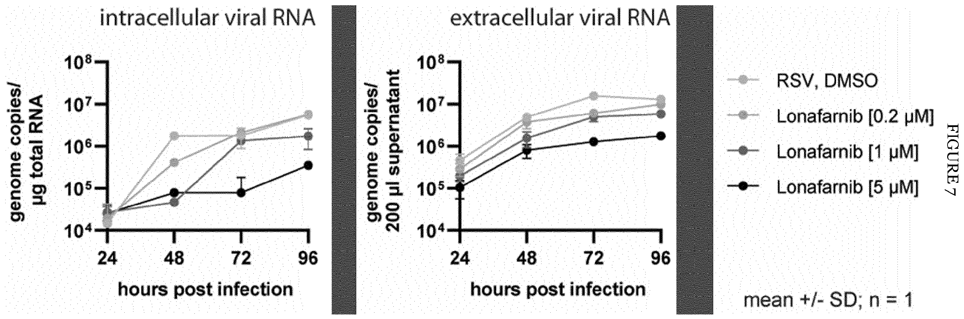

**Figure 7****:** shows that lonafarnib inhibits HRSV infection in differentiated human airway epithelial cells. Shown are viral loads measured by qRT-PCR after HRSV infection in differentiated BCi-NS1.1 cells and treatment with lonafanib at different doses. right: viral load in HBSS rinsing fluid at different time points after infection. left: viral load of harvested cells, also at different time points after infection.

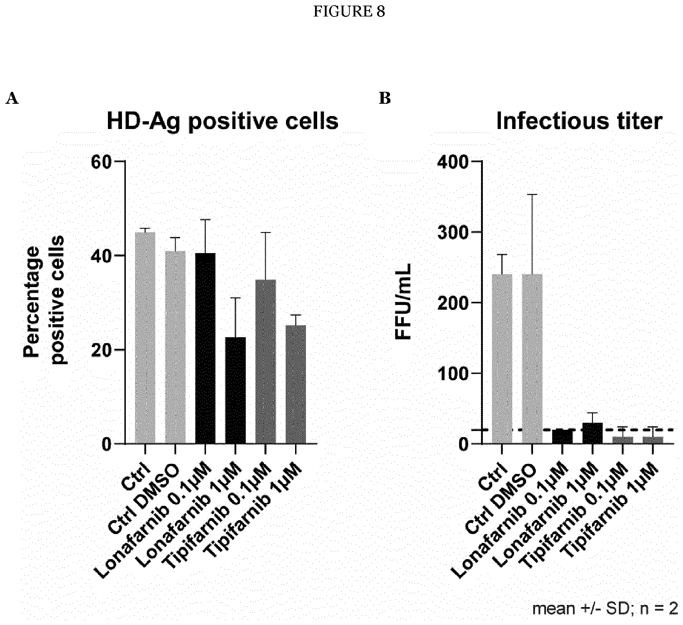

**Figure 8****:** shows that both lonafarnib and tipifarnib efficiently inhibit production of infectious Hepatitis Delta Virus (HDV); Huh7-hNTCP were transfected with 5 ng/ml of plasmids pT7HB2.7 allowing the expression of the HBs-Ag and pSVLD3 containing the HDV RNA sequence. 16h later medium was exchanged and the cells were then cultured in absence or presence of 1% DMSO or increasing concentrations of lonafarnib or tipifarnib; (A) 10 days post transfection, translation of the HDV viral genome in the cells was confirmed by immunofluorescence and percentage of positive cells was calculated; (B) production of infectious progeny HDV was assessed by incubating serial dilutions of their supernatants on naive Huh7-hNTCP, followed by staining for the HD-Ag - dashed lines indicates the detection threshold of the assay.

The sequences show:
**SEQ ID NO 1**: shows the precursor Fusion glycoprotein Fo of Human respiratory syncytial virus A (strain A2) - UniProt identifier P03420 
QNITEEFYQSTCSAVSKGYLSALRTGWYTSVITIELSNIKENKCNGTDAKVKLIKQELDKYKNAV
TELQLLMQSTPPTNNRARRELPRFMNYTLNNAKKTNVTLSKKRKRRFLGFLLGVGSAIASGVA
VSKVLHLEGEVNKIKSALLSTNKAVVSLSNGVSVLTSKVLDLKNYIDKQLLPIVNKQSCSISNIET
VIEFQQKNNRLLEITREFSVNAGVTTPVSTYMLTNSELLSLINDMPITNDQKKLMSNNVQIVRQ
QSYSIMSIIKEEVLAYVVQLPLYGVIDTPCWKLHTSPLCTTNTKEGSNICLTRTDRGWYCDNAGS
VSFFPQAETCKVQSNRVFCDTMNSLTLPSEINLCNVDIFNPKYDCKIMTSKTDVSSSVITSLGAIV
SCYGKTKCTASNKNRGIIKTFSNGCDYVSNK
**SEQ ID NO 2:** shows the precursor Fusion glycoprotein F1 of Human respiratory syncytial virus A (strain A2) - UniProt identifier P03420 
FLGFLLGVGSAIASGVAVSKVLHLEGEVNKIKSALLSTNKAVVSLSNGVSVLTSKVLDLKNYIDK
QLLPIVNKQSCSISNIETVIEFQQKNNRLLEITREFSVNAGVTTPVSTYMLTNSELLSLINDMPIT
NDQKKLMSNNVQIVRQQSYSIMSIIKEEVLAYVVQLPLYGVIDTPCWKLHTSPLCTTNTKEGSNI
CLTRTDRGWYCDNAGSVSFFPQAETCKVQSNRVFCDTMNSLTLPSEINLCNVDIFNPKYDCKIM
TSKTDVSSSVITSLGAIVSCYGKTKCTASNKNRGIIKTFSNGCDYVSNKGMDTVSVGNTLYYVNK
QEGKSLYVKGEPIINFYDPLVFPSDEFDASISQVNEKINQSLAFIRKSDELLHNVNAGKSTTNIMI
TTIIIVIIVILLSLIAVGLLLYCKARSTPVTLSKDQLSGINNIAFSN
**SEQ ID NO 3:** shows the precursor Fusion glycoprotein F2 of Human respiratory syncytial virus A (strain A2) - UniProt identifier P03420

QNITEEFYQSTCSAVSKGYLSALRTGWYTSVITIELSNIKENKCNGTDAKVKLIKQELDK
YKNAVTELQLLMQSTPPTNNRARR

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Primary screening of the ReFRAME library with an HRSV A GFP reporter virus

The ReFRAME drug bank contains 12972 compounds that are either already authorized for use in humans or are in, or have been in, at least one of the three phases of clinical trials. HEp2 cells (ATCC number CCL-23) were infected with the HRSV-A-GFP reporter virus together with the respective compounds (final concentration 5 µM). After 48 hours, infection efficiency was determined by GFP signal, while cell viability was determined by MTT assay. (FIG. 1)

### Example 2: Investigation of the inhibition efficiency of lonafarnib in HRSV infected cells in comparison with farnesyltransferase inhibitor tipifarnib

Efficiency of HRSV infection-inhibition by lonafarnib was investigated by a series of infection assays. In the first round of infection, the effect of lonafarnib on the virus' cell entry, which includes RNA transcription, translation and replication up to 24 hours after infection, was investigated. In the second round of infection, the cell culture supernatant was harvested and transferred to naive cells at 24 h after primary infection. In these cells, again 24 h later, the infection efficiency was determined using the luciferase reporter. In this way, the efficiency was analyzed over a longer period of time, including the spread of the virus and the re-infection of cells. The IC50 values were determined at 0.02 µM (round 1) and 0.004 µM (round 2). (FIG. 2)

In a further trial, the effect of lonafarnib compared to the farnesyltransferase inhibitor tipifarnib on HRSV-infected cell culture was then analyzed and compared. While lonafarnib was highly effective in suppressing HRSV infection, tipifarnib was only slightly effective. (FIG. 3) These findings suggest that lonafarnib does not interfere with HRSV infection by blocking cellular farnesyltransferases.

To confirm the antiviral effect of lonafarnib, an infection assay was performed with the clinical HRSV isolate HRSV H01 ON1. The infection efficiency was assessed in the plaque reduction test, i.e. independently of an indirect reporter gene measurement. Lonafarnib reduced plaque size and number in a dose-dependent manner. (FIG. 4)

Another infection assay was carried out with the HRSV H01 ON1 isolate. The analysis/quantification of the viral infection efficiency was carried out by intracellular FACS staining against a virus own protein. It was shown, that lonafarnib reduced the number of infected cells in a dose-dependent manner. The amount of viral proteins in the infected cells was not reduced by the administration of lonafarnib. (FIG. 5) These results show that lonafarnib inhibits cell entry, but has little effect on RNA replication, transcription and translation of viral proteins after a cell is productively infected.

Further, the efficacy of lonafarnib on HRSV virus population that had previously developed resistance to an HRSV fusion inhibitor was investigated. The effectiveness of HRSV infection (number of infected cells) was determined by intracellular FACS staining. Lonafarnib showed reduced activity in a population of HRSV viruses that had previously developed resistance to an unrelated HRSV fusion inhibitor. The HRSV virus population with resistance to a fusion inhibitor was also less susceptible to inhibition by lonafarnib. (FIG. 6) These data show that lonafarnib acts on the cell entry of HRSV and since the resistance mutations are located in the viral F protein, these data further show that lonafarnib affects the function of the viral F protein and that mutations in the F protein may mediate resistance to lonafarnib.

Then, the antiviral effect of lonafarnib against HRSV was shown in differentiated human airway epithelial cells. Therefore, BCi NS1.1 cells were differentiated and used in so-called "air-liquid-interface" cultures to form a pseudo-stratified lung epithelium. The cells were then infected with HRSV and treated with lonafarnib at different concentrations. The compounds were added to the medium from the basolateral pole of the cell ("liquid interface", homologous to the blood stream) as well as administered apically (homologous to local treatment via inhalation). At different time points post infection, the cell surface ("air interface", apical side of the cell culture, homologous to the lumen of the lung) was rinsed with HBSS to remove released viruses from the cell surface. The viral load in the HBSS rinsing fluid was determined by qRT-PCR. Also, at each time point cells were lysed and the virus load in the cell extract was determined by qRT-PCR. (FIG. 7)

### Example 3: Investigation of the mechanism of lonafarnib in HRSV infection in comparison with farnesyltransferase inhibitor tipifarnib

Figure 8 shows an assay were the antiviral activity of lonafarnib and tipifarnib as farnesyltransferase inhibitors is tested. Both compounds equally inhibit Hepatitis Delta infections, which is known to be mediated by their farnesyltransferase inhibitory activity. Hence, since tipifarnib is not effective against HHRSV, the antiviral mechanism of lonafarnib seems independent of farnesyltransferase inhibition.

### REFERENCES

The references are:
1. Nair, H. et al. Lancet 2010; 375:1545-55
2. see Table 1.1. from the dissertation of Dr. Sake, as well as Cockerill GS. J.Med.Chem. 2019; 62(7):3206-3227

## Claims

1. **A compound for use in the treatment or prevention of a viral disease** in a subject, wherein the viral disease is caused by a virus comprising a membrane fusion glycoprotein (F-protein), wherein the compound is lonafarnib or a derivative thereof, or solvates, salts, stereoisomers, complexes, polymorphs, crystalline forms, racemic mixtures, diastereomers, enantiomers, tautomers, isotopically labelled forms, prodrugs, and combinations thereof.

2. The compound for use of claim 1, wherein the treatment involves inhibition of an F-protein mediated entry of the virus into a host cell.

3. The compound for use of claim 1 or 2, wherein the virus is a human respiratory syncytial virus (HRSV).

4. The compound for use of any one of claims 1 to 3, wherein the viral disease is an HRSV infection of the lower and/or upper respiratory tract in a subject.

5. The compound for use of any one of claims 1 to 4, wherein the subject belongs to one or more risk group indicated for a fatal disease course selected from
(i) Children between 0 and 10 years of age;
(ii) Elderly of 50 years of age or older;
(iii) Immunosuppressed patients.

6. The compound for use of any one of claims 1 to 5, wherein the treatment comprises the administration of one or more additional therapeutic compound selected from antiviral compounds and compounds ameliorating one or more symptoms of the viral disease.

7. The compound for use of claim 6, wherein the antiviral compound is selected from ribavirin, Palivizumab, MK-1654, MEDI8897, JNJ-53718678, Ziresovir, RV521, Lumicitabine and EDP-938, and preferably is Palivizumab.

8. The compound for use of any one of claims 1 to 7, wherein the virus is a HRSV with a resistance to treatment with Palivizumab.

9. The compound for use of any one of claims 1 to 8, wherein the treatment does not involve inhibition of farnesyl transferases, and/or is independent of farnesyl transferase activity.

10. The compound for use of any one of claims 1 to 9, wherein the viral disease is not treatable by inhibition of a farnesyltransferase, and preferably wherein the viral disease is not hepatitis D and Severe Acute Respiratory Syndrome (SARS), such as SARS-CoV2.

11. The compound for use of any one of claims 1 to 10, wherein the compound is administered orally, intravenously or respiratory, for example by oral or nasal inhalation.

12. **A pharmaceutical composition for use in the treatment of a viral disease** in a subject, wherein the viral disease is caused by a virus comprising a membrane fusion glycoprotein (F-protein), wherein the pharmaceutical composition comprises lonafarnib or a derivative thereof, or solvates, salts, stereoisomers, complexes, polymorphs, crystalline forms, racemic mixtures, diastereomers, enantiomers, tautomers, isotopically labelled forms, prodrugs, and combinations thereof, and a pharmaceutically acceptable carrier and/or excipient.

13. **A method for inhibiting entry of a virus into a host cell,** comprising contacting the virus with lonafarnib or a derivative thereof.

14. The method of claim 13, wherein the virus comprises a F-protein, preferably an F-protein comprising an amino acid sequence that is at least 80% identical to a sequence shown in any of SEQ ID NO: 1 to 3.

15. The method of claim 13 or 14, which is an in-vivo or ex-vivo (in-vitro) method.


SEQUENCE LISTING

<110> TWINCORE Zentrum für Experimentelle und Klinische
      Infektionsforschung GmbH
      Medizinische Hochschule Hannover

<120> LONAFARNIB FOR USE IN THE TREATMENT OF VIRAL INFECTIONS

<130> T32506EP

<160> 3

<170> PatentIn version 3.5

<210> 1
<211> 420
<212> PRT
<213> Human respiratory syncytial virus A (strain A2)

<400> 1

Gln Asn Ile Thr Glu Glu Phe Tyr Gln Ser Thr Cys Ser Ala Val Ser
1               5                   10                  15

Lys Gly Tyr Leu Ser Ala Leu Arg Thr Gly Trp Tyr Thr Ser Val Ile
            20                  25                  30

Thr Ile Glu Leu Ser Asn Ile Lys Glu Asn Lys Cys Asn Gly Thr Asp
        35                  40                  45

Ala Lys Val Lys Leu Ile Lys Gln Glu Leu Asp Lys Tyr Lys Asn Ala
    50                  55                  60

Val Thr Glu Leu Gln Leu Leu Met Gln Ser Thr Pro Pro Thr Asn Asn
65                  70                  75                  80

Arg Ala Arg Arg Glu Leu Pro Arg Phe Met Asn Tyr Thr Leu Asn Asn
                85                  90                  95

Ala Lys Lys Thr Asn Val Thr Leu Ser Lys Lys Arg Lys Arg Arg Phe
            100                 105                 110

Leu Gly Phe Leu Leu Gly Val Gly Ser Ala Ile Ala Ser Gly Val Ala
        115                 120                 125

Val Ser Lys Val Leu His Leu Glu Gly Glu Val Asn Lys Ile Lys Ser
    130                 135                 140

Ala Leu Leu Ser Thr Asn Lys Ala Val Val Ser Leu Ser Asn Gly Val
145                 150                 155                 160

Ser Val Leu Thr Ser Lys Val Leu Asp Leu Lys Asn Tyr Ile Asp Lys
                165                 170                 175

Gln Leu Leu Pro Ile Val Asn Lys Gln Ser Cys Ser Ile Ser Asn Ile
            180                 185                 190

Glu Thr Val Ile Glu Phe Gln Gln Lys Asn Asn Arg Leu Leu Glu Ile
        195                 200                 205

Thr Arg Glu Phe Ser Val Asn Ala Gly Val Thr Thr Pro Val Ser Thr
    210                 215                 220

Tyr Met Leu Thr Asn Ser Glu Leu Leu Ser Leu Ile Asn Asp Met Pro
225                 230                 235                 240

Ile Thr Asn Asp Gln Lys Lys Leu Met Ser Asn Asn Val Gln Ile Val
                245                 250                 255

Arg Gln Gln Ser Tyr Ser Ile Met Ser Ile Ile Lys Glu Glu Val Leu
            260                 265                 270

Ala Tyr Val Val Gln Leu Pro Leu Tyr Gly Val Ile Asp Thr Pro Cys
        275                 280                 285

Trp Lys Leu His Thr Ser Pro Leu Cys Thr Thr Asn Thr Lys Glu Gly
    290                 295                 300

Ser Asn Ile Cys Leu Thr Arg Thr Asp Arg Gly Trp Tyr Cys Asp Asn
305                 310                 315                 320

Ala Gly Ser Val Ser Phe Phe Pro Gln Ala Glu Thr Cys Lys Val Gln
                325                 330                 335

Ser Asn Arg Val Phe Cys Asp Thr Met Asn Ser Leu Thr Leu Pro Ser
            340                 345                 350

Glu Ile Asn Leu Cys Asn Val Asp Ile Phe Asn Pro Lys Tyr Asp Cys
        355                 360                 365

Lys Ile Met Thr Ser Lys Thr Asp Val Ser Ser Ser Val Ile Thr Ser
    370                 375                 380

Leu Gly Ala Ile Val Ser Cys Tyr Gly Lys Thr Lys Cys Thr Ala Ser
385                 390                 395                 400

Asn Lys Asn Arg Gly Ile Ile Lys Thr Phe Ser Asn Gly Cys Asp Tyr
                405                 410                 415

Val Ser Asn Lys

            420

<210>  2
<211>  438
<212>  PRT
<213>  Human respiratory syncytial virus A (strain A2)

<400>  2

Phe Leu Gly Phe Leu Leu Gly Val Gly Ser Ala Ile Ala Ser Gly Val
1               5                   10                  15

Ala Val Ser Lys Val Leu His Leu Glu Gly Glu Val Asn Lys Ile Lys
            20                  25                  30

Ser Ala Leu Leu Ser Thr Asn Lys Ala Val Val Ser Leu Ser Asn Gly
        35                  40                  45

Val Ser Val Leu Thr Ser Lys Val Leu Asp Leu Lys Asn Tyr Ile Asp
    50                  55                  60

Lys Gln Leu Leu Pro Ile Val Asn Lys Gln Ser Cys Ser Ile Ser Asn
65                  70                  75                  80

Ile Glu Thr Val Ile Glu Phe Gln Gln Lys Asn Asn Arg Leu Leu Glu
                85                  90                  95

Ile Thr Arg Glu Phe Ser Val Asn Ala Gly Val Thr Thr Pro Val Ser
            100                 105                 110

Thr Tyr Met Leu Thr Asn Ser Glu Leu Leu Ser Leu Ile Asn Asp Met
        115                 120                 125

Pro Ile Thr Asn Asp Gln Lys Lys Leu Met Ser Asn Asn Val Gln Ile
    130                 135                 140

Val Arg Gln Gln Ser Tyr Ser Ile Met Ser Ile Ile Lys Glu Glu Val
145                 150                 155                 160

Leu Ala Tyr Val Val Gln Leu Pro Leu Tyr Gly Val Ile Asp Thr Pro
                165                 170                 175

Cys Trp Lys Leu His Thr Ser Pro Leu Cys Thr Thr Asn Thr Lys Glu
            180                 185                 190

Gly Ser Asn Ile Cys Leu Thr Arg Thr Asp Arg Gly Trp Tyr Cys Asp
        195                 200                 205

Asn Ala Gly Ser Val Ser Phe Phe Pro Gln Ala Glu Thr Cys Lys Val

210                 215                 220

Gln Ser Asn Arg Val Phe Cys Asp Thr Met Asn Ser Leu Thr Leu Pro
225                 230                 235                 240

Ser Glu Ile Asn Leu Cys Asn Val Asp Ile Phe Asn Pro Lys Tyr Asp
                245                 250                 255

Cys Lys Ile Met Thr Ser Lys Thr Asp Val Ser Ser Ser Val Ile Thr
            260                 265                 270

Ser Leu Gly Ala Ile Val Ser Cys Tyr Gly Lys Thr Lys Cys Thr Ala
        275                 280                 285

Ser Asn Lys Asn Arg Gly Ile Ile Lys Thr Phe Ser Asn Gly Cys Asp
    290                 295                 300

Tyr Val Ser Asn Lys Gly Met Asp Thr Val Ser Val Gly Asn Thr Leu
305                 310                 315                 320

Tyr Tyr Val Asn Lys Gln Glu Gly Lys Ser Leu Tyr Val Lys Gly Glu
                325                 330                 335

Pro Ile Ile Asn Phe Tyr Asp Pro Leu Val Phe Pro Ser Asp Glu Phe
            340                 345                 350

Asp Ala Ser Ile Ser Gln Val Asn Glu Lys Ile Asn Gln Ser Leu Ala
        355                 360                 365

Phe Ile Arg Lys Ser Asp Glu Leu Leu His Asn Val Asn Ala Gly Lys
    370                 375                 380

Ser Thr Thr Asn Ile Met Ile Thr Thr Ile Ile Ile Val Ile Ile Val
385                 390                 395                 400

Ile Leu Leu Ser Leu Ile Ala Val Gly Leu Leu Leu Tyr Cys Lys Ala
                405                 410                 415

Arg Ser Thr Pro Val Thr Leu Ser Lys Asp Gln Leu Ser Gly Ile Asn
            420                 425                 430

Asn Ile Ala Phe Ser Asn
        435

<210>  3
<211>  84
<212>  PRT
<213>  Human respiratory syncytial virus A (strain A2)

<400>  3

Gln Asn Ile Thr Glu Glu Phe Tyr Gln Ser Thr Cys Ser Ala Val Ser
1               5                   10                  15

Lys Gly Tyr Leu Ser Ala Leu Arg Thr Gly Trp Tyr Thr Ser Val Ile
            20                  25                  30

Thr Ile Glu Leu Ser Asn Ile Lys Glu Asn Lys Cys Asn Gly Thr Asp
        35                  40                  45

Ala Lys Val Lys Leu Ile Lys Gln Glu Leu Asp Lys Tyr Lys Asn Ala
    50                  55                  60

Val Thr Glu Leu Gln Leu Leu Met Gln Ser Thr Pro Pro Thr Asn Asn
65                  70                  75                  80

Arg Ala Arg Arg